# EUROPÄISCHE PATENTSCHRIFT

(11) **EP 1 824 803 B1**
(45) Veröffentlichungstag und Bekanntmachung des Hinweises auf die Patenterteilung: **10.06.2009**
(21) Anmeldenummer: 05817170.3
(22) Anmeldetag: 08.12.2005
(51) Int. Cl.: C07C 5/327, C07C 7/11, C07C 11/06

(54) **VERFAHREN ZUR HERSTELLUNG VON PROPEN AUS PROPAN**
METHOD FOR THE PRODUCTION OF PROPENE FROM PROPANE
PROCEDE DE FABRICATION DE PROPENE A PARTIR DE PROPANE

(30) Priorität: 09.12.2004 DE 102004059355
(43) Veröffentlichungstag der Anmeldung: 29.08.2007
(73) Patentinhaber: BASF SE, 67056 Ludwigshafen (DE)
(72) Erfinder: CRONE, Sven, 67117 Limburgerhof (DE); MACHHAMMER, Otto, 68163 Mannheim (DE); SCHINDLER, Götz-Peter, 68219 Mannheim (DE)
(74) Vertreter: Hörschler, Wolfram Johannes
(86) Internationale Anmeldenummer: PCT/EP2005/013169
(87) Internationale Veröffentlichungsnummer: WO 2006/061227

(56) Entgegenhaltungen:
- EP-A- 0 864 359
- WO-A-02/083615
- DE-A1- 19 530 454
- US-A- 4 886 928
- US-B1- 6 293 999

## Beschreibung

Die Erfindung betrifft ein Verfahren zur Herstellung von Propen aus Propan.

Propen wird großtechnisch durch Dehydrierung von Propan gewonnen.

In dem als UOP-Oleflex-Prozess bekannten Verfahren zur Dehydrierung von Propan zu Propen wird ein Propan enthaltender Einsatzgasstrom auf 600 - 700 °C vorgeheizt und in einem Wanderbett-Dehydrierreaktor an einem Katalysator, der Platin auf Aluminiumoxid enthält, dehydriert, wobei ein überwiegend Propan, Propen und Wasserstoff enthaltender Produktgasstrom erhalten wird. Daneben sind durch Cracken gebildete leicht siedende Kohlenwasserstoffe (Methan, Ethan, Ethen) sowie geringe Mengen Hochsieder (C₄⁺-Kohlenwasserstoffe) in dem Produktgasstrom enthalten. Das Produktgasgemisch wird abgekühlt und mehrstufig verdichtet. Anschließend werden durch Auskondensieren in einer so genannten "Cold Box" die C₂- und C₃-Kohlenwasserstoffe und die Hochsieder von bei der Dehydrierung gebildetem Wasserstoff und Methan abgetrennt. Das flüssige Kohlenwasserstoff-Kondensat wird anschließend destillativ aufgetrennt, wobei in einer ersten Kolonne die C₂-Kohlenwasserstoffe und verbliebenes Methan abgetrennt und in einer zweiten Destillationskolonne der C₃-Kohlenwasserstoffstrom in eine Propenfraktion mit hohem Reinheitsgrad und eine Propanfraktion, welche auch die C₄⁺-Kohlenwasserstoffe enthält, aufgetrennt wird.

Nachteilig an diesem Verfahren ist der Verlust an C₃-Kohlenwasserstoffen durch das Auskondensieren in der "Cold Box". Wegen der großen Mengen von bei der Dehydrierung gebildetem Wasserstoff und aufgrund des Phasengleichgewichts werden mit dem Wasserstoff/Methan-Abgasstrom auch größere Mengen an C₃-Kohlenwasserstoffen ausgetragen, falls nicht bei sehr tiefen Temperaturen auskondensiert wird. So muss bei Temperaturen von -20 bis -120 °C gearbeitet werden, um den Verlust an C₃-Kohlenwasserstoffen zu begrenzen, die mit dem Wasserstoff/Methan-Abgasstrom ausgetragen werden.

Aufgabe der Erfindung ist es, ein verbessertes Verfahren zur Dehydrierung von Propan zu Propen bereitzustellen.

Gelöst wird die Aufgabe durch ein Verfahren zur Herstellung von Propen aus Propan mit den Schritten
A) ein Propan enthaltende Einsatzgasstrom a wird bereitgestellt;
B) der Propan enthaltende Einsatzgasstrom a und ein sauerstoffhaltiger Gasstrom mit einem Sauerstoff-Gehalt von mindestens 50 Vol.-% werden in eine Dehydrierzone eingespeist und Propan wird einer nicht-oxidativen katalytischen, autothermen Dehydrierung zu Propen unterworfen, wobei ein Produktgasstrom b enthaltend Propan, Propen, Methan, Ethan, Ethen, C₄⁺-Kohlenwasserstoffe, Kohlenmonoxid, Kohlendioxid, Wasserdampf und Wasserstoff erhalten wird;
C) der Produktgasstrom b wird abgekühlt und Wasserdampf durch Kondensierenlassen abgetrennt, wobei ein an Wasserdampf abgereicherter Produktgasstrom c erhalten wird;
D) Kohlendioxid wird durch Gaswäsche abgetrennt, wobei ein an Kohlendioxid abgereicherter Produktgasstrom d erhalten wird;
E) der Produktgasstrom d wird abgekühlt und ein flüssiger Kohlenwasserstoffstrom e1 enthaltend Propan, Propen, Methan, Ethan, Ethen und C₄⁺-Kohlenwasserstoffen durch Kondensierenlassen abgetrennt, wobei ein Restgasstrom e2 enthaltend Methan, Wasserstoff und Kohlenmonoxid verbleibt;
F) der flüssige Kohlenwasserstoffstrom e1 wird in eine erste Destillationszone eingespeist und destillativ in einen Strom f1 enthaltend Propan, Propen und die C₄⁺-Kohlenwasserstoffe und einen Strom f2 enthaltend Ethan und Ethen aufgetrennt;
G) der Strom f1 wird in eine zweite Destillationszone eingespeist und destillativ in einen Produktstrom g1 enthaltend Propen und einen Strom g2 enthaltend Propan und die C₄⁺-Kohlenwasserstoffe aufgetrennt.

In einem ersten Verfahrensteil A wird ein Propan enthaltender Einsatzgasstrom a bereitgestellt. Dieser enthält im Allgemeinen mindestens 80 Vol.-% Propan, vorzugsweise 90 Vol.-% Propan. Daneben enthält der propanhaltige Einsatzgasstrom A im Allgemeinen noch Butane (n-Butan, iso-Butan). Typische Zusammensetzungen des propanhaltigen Einsatzgasstroms sind in DE-A 102 46 119 und DE-A 102 45 585 offenbart. Üblicherweise wird der propanhaltige Einsatzgasstrom a aus liquid petroleum gas (LPG) gewonnen.

In einem Verfahrensteil B wird der Propan enthaltende Einsatzgasstrom in eine Dehydrierzone eingespeist und einer nicht-oxidativen katalytischen Dehydrierung unterworfen. Dabei wird Propan in einem Dehydrierreaktor an einem dehydrieraktiven Katalysator teilweise zu Propen dehydriert. Daneben fallen Wasserstoff und in geringen Mengen Methan, Ethan, Ethen und C₄⁺-Kohlenwasserstoffe (n-Butan, iso-Butan, Butene, Butadien) an. Außerdem fallen Kohlenstoffoxide (CO, CO₂), insbesondere CO₂, Wasser und in geringen Umfang Inertgase im Produktgasgemisch der nicht-oxidativen katalytischen, autothermen Propan-Dehydrierung an. Inertgase (Stickstoff) werden mit dem bei der autothermen Dehydrierung eingesetzten Sauerstoffstrom eingebracht, sofern kein reiner Sauerstoff eingespeist wird. Daneben liegt im Produktgasgemisch nicht umgesetztes Propan vor.

Die nicht-oxidative katalytische Propan-Dehydrierung wird autotherm durchgeführt. Dazu wird dem Reaktionsgasgemisch der Propan-Dehydrierung in mindestens einer Reaktionszone zusätzlich Sauerstoff zugemischt und der in dem Reaktionsgasgemisch enthaltene Wasserstoff und/oder Kohlenwasserstoff zumindest teilweise verbrannt, wodurch zumindest ein Teil der benötigten Dehydrierwärme in der mindestens einen Reaktionszone direkt in dem Reaktionsgasgemisch erzeugt wird.

Ein Merkmal der nicht-oxidativen Fahrweise gegenüber einer oxidativen Fahrweise ist die zumindest intermediäre Bildung von Wasserstoff. Bei der oxidativen Dehydrierung wird kein freier Wasserstoff in wesentlichen Mengen gebildet.

Die nicht-oxidative katalytische Propan-Dehydrierung kann grundsätzlich in allen aus dem Stand der Technik bekannten Reaktortypen durchgeführt werden. Eine vergleichsweise ausführliche Beschreibung von erfindungsgemäß geeigneten Reaktortypen enthält auch "Catalytica^{®} Studies Division, Oxidative Dehydrogenation and Alternative Dehydrogenation Processes" (Study Number 4192 OD, 1993, 430 Ferguson Drive, Mountain View, California, 94043-5272, USA).

Eine geeignete Reaktorform ist der Festbettrohr- oder Rohrbündelreaktor. Bei diesen befindet sich der Katalysator (Dehydrierungskatalysator und gegebenenfalls spezieller Oxidationskatalysator) als Festbett in einem Reaktionsrohr oder in einem Bündel von Reaktionsrohren. Übliche Reaktionsrohr-Innendurchmesser betragen etwa 10 bis 15 cm. Ein typischer Dehydrierrohrbündelreaktor umfasst ca. 300 bis 1000 Reaktionsrohre. Die Temperatur im Reaktionsrohrinneren bewegt sich üblicherweise im Bereich von 300 bis 1200°C, vorzugsweise im Bereich von 500 bis 1000°C. Der Arbeitsdruck liegt üblicherweise zwischen 0,5 und 8 bar, häufig zwischen 1 und 2 bar bei Verwendung einer geringen Wasserdampfverdünnung, aber auch zwischen 3 und 8 bar bei Verwendung einer hohen Wasserdampfverdünnung (entsprechend dem so genannten "steam active reforming process" (STAR-Prozess) oder dem Linde-Verfahren) zur Dehydrierung von Propan oder Butan von Phillips Petroleum Co. Typische Katalysatorbelastungen (GHSV) liegen bei 500 bis 2000 h⁻¹, bezogen auf eingesetzten Kohlenwasserstoff. Die Katalysatorgeometrie kann beispielsweise kugelförmig oder zylindrisch (hohl oder voll) sein.

Die nicht-oxidative katalytische, autotherme Propan-Dehydrierung kann auch, entsprechend dem Snamprogetti/Yarsintez-FBD-Prozess, heterogen katalysiert im Wirbelbett durchgeführt werden. Zweckmäßigerweise werden dabei zwei Wirbelbetten nebeneinander betrieben, von denen sich eines in der Regel im Zustand der Regenerierung befindet. Der Arbeitsdruck beträgt typischerweise 1 bis 2 bar, die Dehydriertemperatur in der Regel 550 bis 600°C. Die für die Dehydrierung erforderliche Wärme kann dabei in das Reaktionssystem eingebracht werden, indem der Dehydrierkatalysator auf die Reaktionstemperatur vorerhitzt wird. Durch die Zumischung eines Sauerstoff enthaltenden Co-Feeds kann auf die Vorerhitzer verzichtet werden, und die benötigte Wärme wird direkt im Reaktorsystem durch Verbrennung von Wasserstoff und/oder Kohlenwasserstoffen in Gegenwart von Sauerstoff erzeugt. Gegebenenfalls kann zusätzlich ein Wasserstoff enthaltender Co-Feed zugemischt werden.

Die nicht-oxidative katalytische, autotherme Propan-Dehydrierung wird bevorzugt in einem Hordenreaktor durchgeführt. Dieser enthält ein oder mehrere aufeinander folgende Katalysatorbetten. Die Anzahl der Katalysatorbetten kann 1 bis 20, zweckmäßigerweise 1 bis 6, bevorzugt 1 bis 4 und insbesondere 1 bis 3 betragen. Die Katalysatorbetten werden vorzugsweise radial oder axial vom Reaktionsgas durchströmt. Im Allgemeinen wird ein solcher Hordenreaktor mit einem Katalysatorfestbett betrieben. Im einfachsten Fall sind die Katalysatorfestbetten in einem Schachtofenreaktor axial oder in den Ringspalten von konzentrisch angeordneten zylindrischen Gitterrosten angeordnet. Ein Schachtofenreaktor entspricht einer Horde. Die Durchführung der Dehydrierung in einem einzelnen Schachtofenreaktor entspricht einer Ausführungsform. In einer weiteren bevorzugten Ausführungsform wird die Dehydrierung in einem Hordenreaktor mit 3 Katalysatorbetten durchgeführt.

Im Allgemeinen wird die Menge des dem Reaktionsgasgemisch zugesetzten sauerstoffhaltigen Gases so gewählt, dass durch die Verbrennung von im Reaktionsgasgemisch vorhandenen Wasserstoff und gegebenenfalls von im Reaktionsgasgemisch vorliegenden Kohlenwasserstoffen und/oder von in Form von Koks vorliegendem Kohlenstoff die für die Dehydrierung des Propans benötigte Wärmemenge erzeugt wird. Im Allgemeinen beträgt die insgesamt zugeführte Sauerstoffmenge, bezogen auf die Gesamtmenge des Propans, 0,001 bis 0,5 mol/mol, bevorzugt 0,005 bis 0,25 mol/mol, besonders bevorzugt 0,05 bis 0,25 mol/mol. Sauerstoff kann entweder als reiner Sauerstoff oder als sauerstoffhaltiges Gas, welches Inertgase enthält, eingesetzt werden. Um hohe Propan- und Propen-Verluste bei der Aufarbeitung (siehe unten) zu vermeiden ist es jedoch wesentlich, dass der Sauerstoff-Gehalt des eingesetzten sauerstoffhaltigen Gases hoch ist und mindestens 50 Vol.-%, bevorzugt mindestens 80 Vol.-%, besonders bevorzugt mindestens 90 Vol.-% beträgt. Besonders bevorzugtes sauerstoffhaltiges Gas ist technisch reiner Sauerstoff mit einem O₂-Gehalt von ca. 99 Vol.%.

Der zur Wärmeerzeugung verbrannte Wasserstoff ist der bei der katalytischen Propan-Dehydrierung gebildete Wasserstoff sowie gegebenenfalls dem Reaktionsgasgemisch als wasserstoffhaltiges Gas zusätzlich zugesetzter Wasserstoff. Vorzugsweise sollte soviel Wasserstoff zugegen sein, dass das Molverhältnis H₂/O₂ im Reaktionsgasgemisch unmittelbar nach der Einspeisung von Sauerstoff 1 bis 10, bevorzugt 2 bis 5 mol/mol beträgt. Dies gilt bei mehrstufigen Reaktoren für jede Zwischeneinspeisung von sauerstoffhaltigem und gegebenenfalls wasserstoffhaltigem Gas.

Die Wasserstoffverbrennung erfolgt katalytisch. Der eingesetzte Dehydrierungskatalysator katalysiert im Allgemeinen auch die Verbrennung der Kohlenwasserstoffe und von Wasserstoff mit Sauerstoff, so dass grundsätzlich kein von diesem verschiedener spezieller Oxidationskatalysator erforderlich ist. In einer Ausführungsform wird in Gegenwart eines oder mehrerer Oxidationskatalysatoren gearbeitet, die selektiv die Verbrennung von Wasserstoff mit Sauerstoff in Gegenwart von Kohlenwasserstoffen katalysieren. Die Verbrennung dieser Kohlenwasserstoffe mit Sauerstoff zu CO, CO₂ und Wasser läuft dadurch nur in untergeordnetem Maße ab. Vorzugsweise liegen der Dehydrierungskatalysator und der Oxidationskatalysator in verschiedenen Reaktionszonen vor.

Bei mehrstufiger Reaktionsführung kann der Oxidationskatalysator in nur einer, in mehreren oder in allen Reaktionszonen vorliegen.

Bevorzugt ist der Katalysator, der selektiv die Oxidation von Wasserstoff katalysiert, an den Stellen angeordnet, an denen höhere Sauerstoffpartialdrucke herrschen als an anderen Stellen des Reaktors, insbesondere in der Nähe der Einspeisungsstelle für das sauerstoffhaltige Gas. Die Einspeisung von sauerstoffhaltigem Gas und/oder wasserstoffhaltigem Gas kann an einer oder mehreren Stelle des Reaktors erfolgen.

In einer Ausführungsform des erfindungsgemäßen Verfahrens erfolgt eine Zwischeneinspeisung von sauerstoffhaltigem Gas und von wasserstoffhaltigem Gas vor jeder Horde eines Hordenreaktors. In einer weiteren Ausführungsform des erfindungsgemäßen Verfahrens erfolgt die Einspeisung von sauerstoffhaltigem Gas und von wasserstoffhaltigem Gas vor jeder Horde außer der ersten Horde. In einer Ausführungsform ist hinter jeder Einspeisungsstelle eine Schicht aus einem speziellen Oxidationskatalysator vorhanden, gefolgt von einer Schicht aus dem Dehydrierungskatalysator. In einer weiteren Ausführungsform ist kein spezieller Oxidationskatalysator vorhanden. Die Dehydriertemperatur beträgt im allgemeinen 400 bis 1100 °C, der Druck im letzten Katalysatorbett des Hordenreaktors im Allgemeinen 0,2 bis 5 bar, bevorzugt 1 bis 3 bar. Die Belastung (GHSV) beträgt im allgemeinen 500 bis 2000 h⁻¹, bei Hochlastfahrweise auch bis zu 100 000 h⁻¹, bevorzugt 4000 bis 16 000 h⁻¹.

Ein bevorzugter Katalysator, der selektiv die Verbrennung von Wasserstoff katalysiert, enthält Oxide und/oder Phosphate, ausgewählt aus der Gruppe bestehend aus den Oxiden und/oder Phosphaten von Germanium, Zinn, Blei, Arsen, Antimon oder Bismut. Ein weiterer bevorzugter Katalysator, der die Verbrennung von Wasserstoff katalysiert, enthält ein Edelmetall der VIII. und/oder I. Nebengruppe.

Die eingesetzten Dehydrierungskatalysatoren weisen im Allgemeinen einen Träger und eine Aktivmasse auf. Der Träger besteht dabei in der Regel aus einem wärmebeständigen Oxid oder Mischoxid. Bevorzugt enthalten die Dehydrierungskatalysatoren ein Metalloxid, das ausgewählt ist aus der Gruppe bestehend aus Zirkondioxid, Zinkoxid, Aluminiumoxid, Siliciumdioxid, Titandioxid, Magnesiumoxid, Lanthanoxid, Ceroxid und deren Gemischen, als Träger. Bei den Gemischen kann es sich um physikalische Mischungen oder auch um chemische Mischphasen wie Magnesium- oder Zinkaluminiumoxid-Mischoxide handeln. Bevorzugte Träger sind Zirkondioxid und/oder Siliziumdioxid, besonders bevorzugt sind Gemische aus Zirkondioxid und Siliziumdioxid.

Die Aktivmasse der Dehydrierungskatalysatoren enthalten im allgemeinen ein oder mehrere Elemente der VIII. Nebengruppe, bevorzugt Platin und/oder Palladium, besonders bevorzugt Platin. Darüber hinaus können die Dehydrierungskatalysatoren ein oder mehrere Elemente der I. und/oder II. Hauptgruppe aufweisen, bevorzugt Kalium und/oder Cäsium. Weiterhin können die Dehydrierungskatalysatoren ein oder mehrere Elemente der III. Nebengruppe einschließlich der Lanthaniden und Actiniden enthalten, bevorzugt Lanthan und/oder Cer. Schließlich können die Dehydrierungskatalysatoren ein oder mehrere Elemente der III. und/oder IV. Hauptgruppe aufweisen, bevorzugt ein oder mehrere Elemente aus der Gruppe bestehend aus Bor, Gallium, Silizium, Germanium, Zinn und Blei, besonders bevorzugt Zinn.

In einer bevorzugten Ausführungsform enthält der Dehydrierungskatalysator mindestens ein Element der VIII. Nebengruppe, mindestens ein Element der I. und/oder II. Hauptgruppe, mindestens ein Element der III. und/oder IV. Hauptgruppe und mindestens ein Element der III. Nebengruppe einschließlich der Lanthaniden und Actiniden.

Beispielsweise können erfindungsgemäß alle Dehydrierkatalysatoren eingesetzt werden, die in den WO 99/46039, US 4,788,371, EP-A 705 136, WO 99/29420, US 5,220,091, US 5,430,220, US 5,877,369, EP 0 117 146, DE-A 199 37 106, DE-A 199 37 105 und DE-A 199 37 107 offenbart werden. Besonders bevorzugte Katalysatoren für die vorstehend beschriebenen Varianten der autothermen Propan-Dehydrierung sind die Katalysatoren gemäß den Beispiel 1, 2, 3 und 4 der DE-A 199 37 107.

Die autotherme Propan-Dehydrierung wird bevorzugt in Gegenwart von Wasserdampf durchgeführt. Der zugesetzte Wasserdampf dient als Wärmeträger und unterstützt die Vergasung von organischen Ablagerungen auf den Katalysatoren, wodurch der Verkokung der Katalysatoren entgegengewirkt und die Standzeit der Katalysatoren erhöht wird. Dabei werden die organischen Ablagerungen in Kohlenmonoxid, Kohlendioxid und gegebenenfalls Wasser umgewandelt.

Der Dehydrierungskatalysator kann in an sich bekannter Weise regeneriert werden. So kann dem Reaktionsgasgemisch Wasserdampf zugesetzt werden oder von Zeit zu Zeit ein Sauerstoff enthaltendes Gas bei erhöhter Temperatur über die Katalysatorschüttung geleitet werden und der abgeschiedene Kohlenstoff abgebrannt werden. Durch die Verdünnung mit Wasserdampf wird das Gleichgewicht zu den Produkten der Dehydrierung verschoben. Gegebenenfalls wird der Katalysator nach der Regenerierung mit einem wasserstoffhaltigen Gas reduziert.

Bei der autothermen Propan-Dehydrierung wird ein Gasgemisch erhalten, welches im Allgemeinen folgende Zusammensetzung aufweist: 10 bis 45 Vol.-% Propan, 5 bis 40 Vor.-% Propen, 0 bis 5 Vol.-% Methan, Ethan, Ethen und C₄⁺-Kohlenwasserstoffe, 0 bis 5 Vol.-% Kohlendioxid, 0 bis 20 Vol.-% Wasserdampf und 0 bis 25 Vol.-% Wasserstoff sowie 0 bis 5 Vol.-% Inertgase.

Der Produktgasstrom b steht beim Verlassen der Dehydrierzone im Allgemeinen unter einem Druck von 1 bis 5 bar, vorzugsweise 1,5 bis 3 bar, und weist eine Temperatur im Bereich von 400 bis 700 °C auf.

Der Produktgasstrom b kann in zwei Teilströme aufgetrennt werden, wobei ein Teilstrom in die autotherme Dehydrierung zurückgeführt wird, entsprechend der in DE-A 102 11 275 und DE-A 100 28 582 beschriebenen Kreisgasfahrweise.

In Verfahrensteil C wird zunächst Wasser aus dem Produktgasstrom b abgetrennt. Die Abtrennung von Wasser wird durch Kondensieren lassen durch Abkühlen und ggf. Verdichten des Produktgasstroms b durchgeführt und kann in einer oder mehreren Abkühlungs- und gegebenenfalls Verdichtungsstufen durchgeführt werden. Im Allgemeinen wird der Produktgasstrom b hierzu auf eine Temperatur im Bereich von 30 bis 80 °C, vorzugsweise 40 bis 65 °C abgekühlt. Zusätzlich kann der Produktgasstrom verdichtet werden, beispielsweise auf einen Druck im Bereich von 5 bis 25 bar.

In einer Ausführungsform des erfindungsgemäßen Verfahrens wird der Produktgasstrom b durch eine Kaskade von Wärmetauschern geleitet und so zunächst auf eine Temperatur im Bereich von 50 bis 200 °C abgekühlt und anschließend in einem Quenchturm mit Wasser auf eine Temperatur von 40 bis 80 °C, beispielsweise 55 °C weiter abgekühlt. Dabei kondensiert der größte Teil des Wasserdampfs aus, aber auch ein Teil der im Produktgasstrom b enthaltenen C₄⁺-Kohlenwasserstoffe, insbesondere die C₅⁺-Kohlenwasserstoffe.

Es wird ein an Wasserdampf abgereicherter Produktgasstrom c erhalten. Dieser enthält im Allgemeinen noch 0 bis 5 Vol.-% Wasserdampf. Zur praktisch vollständigen Entfernung von Wasser aus dem Produktgasstrom c kann eine Trocknung mittels Molekularsieb vorgesehen werden.

In einem Verfahrensschritt D) wird aus dem Produktgasstrom c Kohlendioxid wird durch Gaswäsche abgetrennt, wobei ein an Kohlendioxid abgereicherter Produktgasstrom d erhalten wird. Der Kohlendioxid-Gaswäsche kann eine gesonderte Verbrennungsstufe vorgeschaltet werden, in der Kohlenmonoxid selektiv zu Kohlendioxid oxidiert wird.

Zur CO₂-Abtrennung wird im Allgemeinen Natronlauge, Kalilauge oder eine Alkanolamin-Lösung als Waschflüssigkeit eingesetzt, bevorzugt wird eine aktivierte N-Methyldiethanolamin-Lösung eingesetzt. Im Allgemeinen wird vor Durchführung der Gaswäsche der Produktgasstrom c durch ein- oder mehrstufige Verdichtung auf einen Druck im Bereich von 5 bis 25 bar verdichtet.

Es wird ein an Kohlendioxid abgereicherter Produktgasstrom d mit einem CO₂-Gehalt von im Allgemeinen < 100 ppm, vorzugsweise < 10 ppm erhalten.

In einem Verfahrensschritt E) wird der Produktgasstrom d abgekühlt und ein flüssiger Kohlenwasserstoffstroms e1 enthaltend Propan, Propen, Methan, Ethan, Ethen und C₄⁺-Kohlenwasserstoffen durch Kondensieren lassen abgetrennt, wobei ein Restgasstrom e2 enthaltend Methan, Wasserstoff und Kohlenmonoxid verbleibt. Dazu wird der Produktgasstrom d im Allgemeinen auf einen Druck im Bereich von 5 bis 25 bar verdichtet und auf eine Temperatur im Bereich von -10 bis -120 °C abgekühlt. Die Verdichtung kann mehrstufig erfolgen, beispielsweise in zwei oder drei Stufen, vorzugsweise erfolgt sie mehrstufig, beispielsweise dreistufig. In einer Ausführungsform des erfindungsgemäßen Verfahrens erfolgt vor Durchführung des Waschschritts C) eine ein- oder zweistufige Kompression des Produktgasstroms c auf einen Druck im Bereich von 5 bis 12 bar und anschließend eine ein- oder zweistufige Kompression des Produktgasstroms d auf einen Druck im Bereich von 10 bis 25 bar. Auch die Kühlung kann mehrstufig erfolgen, bevorzugt erfolgt sie mehrstufig. Geeignete Kühlmittel sind Ethen, Propen und Propan, welche durch Verdichten auf Drücke bis zu 20 bar und anschließendes Entspannen auf Temperaturen im Bereich von -40 °C bis -100 °C abgekühlt werden.

Im Allgemeinen erfolgt das Abkühlen des Produktgasstroms durch Wärmetausch mit einem Kühlmittel in einer Kompressionskälteanlage ("Cold Box"). Die Abkühlung kann mehrstufig unter Einsatz mehrerer Kühlkreisläufe erfolgen. Die Abkühlung kann mehrstufig in einer Kolonne erfolgen, wobei das in der Kolonne aufsteigende Gas entnommen, abgekühlt, (teilweise) kondensiert und in die Kolonne zurückgeführt wird. Am Kolonnensumpf wird das Kondensat, am Kolonnenkopf das nicht kondensierte Gas entnommen, welches auch im obersten Kühlkreislauf nicht kondensiert ist.

Durch den geringen Wasserstoffgehalt des Produktgasstroms d als Folge der Durchführung der Dehydrierung B) als autotherme Dehydrierung unter simultaner Verbrennung des gebildeten Wasserstoffs werden in dem Abtrennschritt E) die C₃-Kohlenwasserstoffe ganz überwiegend auskondensiert und wird nur ein sehr kleiner Teil der C₃-Kohlenwasserstoffe mit dem Wasserstoff/Methan-Abgasstrom ausgetragen.

Es wird ein flüssiger Kohlenwasserstoff-Kondensatstrom e1 erhalten, der im Allgemeinen 20 bis 60 mol-% Propan, 20 bis 60 mol% Propen, 0 bis 5 mol-% Methan, 0 bis 5 mol% Ethan und Ethen und 0 bis 5 mol-% C₄⁺-Kohlenwasserstoffe enthält.

Vor Durchführung von Schritt E) wird eine Trocknungsstufe durchgeführt, in der der Produktgasstrom d durch Überleiten über ein Molekularsieb getrocknet wird. Geeignete Molekularsiebe sind dem Fachmann bekannt. Im Allgemeinen wird die Trocknungsstufe nach der letzten Verdichtungsstufe unmittelbar vor dem Kondensationsschritt E) durchgeführt. Die Temperatur des zu trocknenden Produktgasstroms d beträgt im Allgemeinen von 0 bis 50 °C, vorzugsweise 10 bis 30 °C.

Nach Durchführung des Trocknungsschritts beträgt der Wassergehalt des Gasstroms d < 500 ppm, bevorzugt < 100 ppm.

In einem Verfahrensschritt F wird der flüssige Kohlenwasserstoffstrom e1 in eine erste Destillationszone eingespeist und destillativ in einen Strom f1 enthaltend die C₃-Kohlenwasserstoffe Propan, Propen und die C₄⁺-Kohlenwasserstoffe und einen Strom f2 enthaltend die C₂-Kohlenwasserstoffe Ethan und Ethen aufgetrennt. Dazu wird der Kohlenwasserstoffstrom e1 im Allgemeinen in eine C2/C3-Trennkolonne mit typischer Weise 20 bis 60 theoretischen Böden, beispielsweise ca. 30 theoretischen Böden, eingespeist. Diese wird im Allgemeinen bei einem Druck im Bereich von 12 bis 30 bar betrieben, beispielsweise bei ca. 25 bar. Die Sumpftemperatur beträgt im Allgemeinen von 40 bis 100 °C, beispielsweise ca. 60 °C, die Kopftemperatur von -10 bis 10 °C, beispielsweise ca. 10 °C.

Es wird ein Strom f1 aus Propan, Propen und den C₄⁺-Kohlenwasserstoffen als Sumpfabzugsstrom mit einem Ethan/Ethen-Gehalt von im Allgemeinen insgesamt < 5000 ppm, vorzugsweise < 1000 ppm, besonders bevorzugt < 500 ppm erhalten.

In einem Verfahrensschritt G wird der flüssige Kohlenwasserstoffstrom f1 in eine erste Destillationszone eingespeist und destillativ in einen Strom g1 enthaltend Propen und einen Strom g2 enthaltend Propan und die C₄⁺-Kohlenwasserstoffe aufgetrennt. Dazu wird der Kohlenwasserstoffstrom f1 im Allgemeinen in eine C3-Trennkolonne ("C3-Splitter") mit typischer Weise 80 bis 200 theoretischen Böden, beispielsweise ca. 100 theoretischen Böden, eingespeist. Diese wird im Allgemeinen bei einem Druck im Bereich von 15 bis 30 bar betrieben, beispielsweise bei ca. 25 bar. Die Sumpftemperatur beträgt im Allgemeinen von 40 bis 100 °C, beispielsweise ca. 68 °C, die Kopftemperatur von 30 bis 60 °C, beispielsweise ca. 60 °C.

Die Erfindung wird nachstehend unter Bezugnahme auf die Zeichnung näher erläutert.

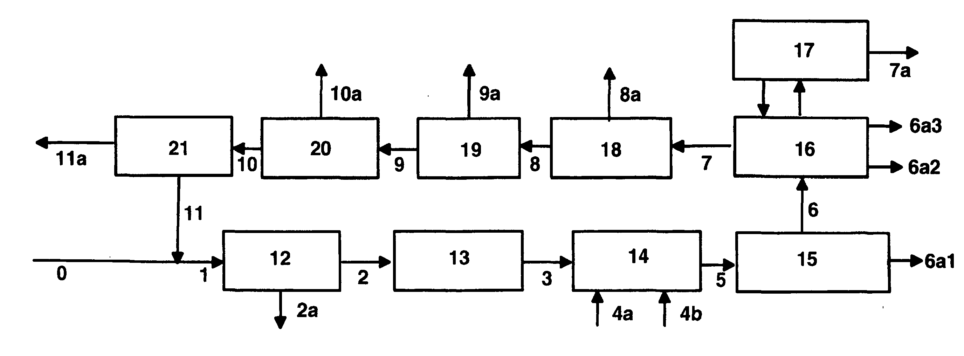

Figur 1 zeigt schematisch eine Ausführungsform des erfindungsgemäßen Verfahrens.

Frisches Propan 0, welches beispielsweise zu 98 Vol.-% Propan besteht und daneben 2 Vol.-% C₄⁺-Kohlenwasserstoffe (hauptsächlich Butan) enthält, wird mit dem Rückführstrom aus der C3-Auftrennkolonne 11 aus Propan und Propen in eine C3/C4-Auftrennkolonne 12 eingespeist und destillativ in einen Strom 2a aus C₄⁺-Kohlenwasserstoffen 2a, welcher als Sumpfabzugsstrom erhalten wird, und einen Strom 2 mit einem Propan-Gehalt von > 99 Vol.-%, der als Kopfabzugsstrom erhalten wird, aufgetrennt.

Der Propanstrom 2 wird in einem Vorerhitzer 13, beispielsweise durch Wärmetausch mit dem Produktgasstrom 5, auf eine Temperatur von ca. 450°C vorerhitzt und tritt von dort mit dieser Temperatur in den Dehydrierreaktor 14 ein. Der Dehydrierreaktor 3 ist vorzugsweise als Radialstromreaktor ausgebildet. In diesen werden zusätzlich ein Wasserdampfstrom 4a und ein Sauerstoffstrom 4b aus technisch reinem Sauerstoff mit einem Sauerstoffgehalt von ca. 99 Vol.-% eingespeist. Das Produktgasgemisch 5 verlässt den Reaktor mit einer Temperatur von ca. 600°C und besteht aus Propan, Propen, Methan, Ethan, Ethen, C₄-Kohlenwasserstoffen, Kohlendioxid, Wasserdampf und Wasserstoff sowie geringen Mengen an Stickstoff. Der Produktgasstrom 3 wird in einer Kühlerkaskade auf eine Temperatur von ca. 55°C abgekühlt. In der Kühlerkaskade 6 kondensiert der größte Teil des Wasserdampfs aus und wird als flüssiges Kondensat 6a1 abgezogen.

Der Strom 6 wird in einem dreistufigen Verdichter mit Zwischenkühlung von ca. 2 bar auf ca. 15 bar verdichtet. Dabei kondensieren weitere Mengen Wasserdampf aus und werden als flüssiges Kondensat 6a2 und 6a3 abgezogen. Zwischen der zweiten und dritten Verdichterstufe erfolgt die Abtrennung von Kohlendioxid mittels Gaswäsche. Hierzu wird der verdichtete Gasstrom in eine Waschkolonne 17 eingeleitet und mit einer aktivierten N-Methyldiethanolamin-Lösung als Waschflüssigkeit in Kontakt gebracht. Aus der beladenen Waschflüssigkeit wird in einer Desorptionskolonne durch Erwärmen Kohlendioxid wieder freigesetzt und die Waschflüssigkeit regeneriert.

Der auf ca. 15 bar verdichtete und von Kohlendioxid befreite Gasstrom 7 wird auf ca. 10 °C vorgekühlt, einer Trocknungsstufe 18 zugeführt und dort mittels eines Molsiebs von Wasserspuren befreit. Hierzu werden 2 parallele, mit Molsieben gefüllte Apparate eingesetzt, von denen stets einer im Absorptionsmodus und der andere im Regenerationsmodus betrieben wird. Der getrocknete Gasstrom 8 tritt mit einer Temperatur von ca. 10 °C in die "Cold Box" ein, wo bei -40°C Ethan, Ethen, Propan, Propen, die C₄⁺-Kohlenwasserstoffe und ein Teil des Methans als flüssiges Kohlenwasserstoffgemisch 9 auskondensieren. Dabei verbleibt ein Abgasstrom 9a, welcher einen geringen Teil der in dem Gasstrom 8 enthaltenen C₂-Kohlenwasserstoffe, den überwiegenden Teil des im Gasstrom 8 enthaltenen Methans und praktisch vollständig den im Gasstrom 8 enthaltenen Wasserstoff enthält.

Das flüssige Kohlenwasserstoffgemisch 9 wird in einer C2/C3-Auftrennkolonne 20 destillativ in einen Strom 10 aus Propan, Propen und C₄⁺-Kohlenwasserstoffen und einen Strom 10a aus Methan, Ethan und Ethen aufgetrennt. Der Strom 10 wird einer C3-Auftrennkolonne 21 zugeführt und in einen Produktstrom 11a aus Propen mit einem Reinheitsgrad von 99,5 Gew.-% und den Rückführstrom 11, der überwiegend aus Propan besteht und daneben noch C₄⁺-Kohlenwasserstoffe sowie geringe Mengen an Propen (ca. 1 Gew.-%) enthält, aufgetrennt.

Die Erfindung wird durch das nachstehende Beispiel näher erläutert.

### Beispiele

Die in der Figur dargestellte Variante des erfindungsgemäßen Verfahrens wurde rechnerisch simuliert. Dabei wurden die nachfolgenden Verfahrensparameter angenommen.

### Beispiel 1

Es wird eine Kapazität der Anlage von 350 kt/a bei 8000 h Laufzeit, entsprechend 43750 kg/h Propen, angenommen.

Das Firsch-Propan enthält neben 98 Gew.-% Propan 2 Gew.-% Butan. Der Butangehalt wird in einer C3/C4-Trennkolonne mit 40 theoretischen Stufen bei 10 bar Betriebsdruck und einem Rücklaufverhältnis von 0,41 auf 0,01 Gew.-% abgereichert.

Der Propanstrom wird auf 450°C vorgeheizt, tritt in die Dehydrierzone ein und wird einer autothermen Dehydrierung unterworfen. Der Umsatz der Dehydrierung beträgt, bezogen auf Propan, 50%, die Selektivität der Propen-Bildung beträgt 90%. Es werden daneben 5% Crack-Produkte und 5% CO₂ durch Totalverbrennung gebildet. Die Wasserkonzentration im Austrittsgas der Dehydrierzone beträgt 9 Gew.%, der Restsauerstoffgehalt im Austrittsgas beträgt 0 Gew.%, die Austrittstemperatur des Pröduktgasgemischs beträgt 600°C.

Das Austrittsgas wird bei 1,8 bar auf 55°C abgekühlt und Wasser bis zum Sättigungsdampfdruck auskondensiert.

Anschließend wird das Produktgasgemisch 3-stufig verdichtet. In der ersten Verdichterstufe wird von 1,8 bar auf 4,5 bar, in der zweiten Verdichterstufe von 4,5 auf 11 bar und in der dritten Verdichterstufe von 11 bar auf 18 bar verdichtet. Nach jeder Verdichterstufe wird das Gasgemisch auf 55 °C abgekühlt. Nach der zweiten Verdichterstufe wird CO₂ durch Gaswäsche vollständig aus dem Gasstrom abgetrennt. Nach der dritten Verdichterstufe wird das Restwasser vollständig aus dem Gasstrom abgetrennt.

Anschließend wird der Gasstrom bei 18 bar auf -40°C abgekühlt. Dabei kondensieren die C₂⁻, C₃⁻ und C₄⁺-Kohlenwasserstoffe aus.

Das flüssige Kohlenwasserstoff-Kondensat wird in einer C2/C3-Trennkolonne mit 30 theoretischen Stufen bei 25 bar und einem Rücklaufverhältnis von 0,74 aufgetrennt. Die Sumpftemperatur beträgt 62 °C, die Kopftemperatur 10 °C. Der Rest-Ethangehalt des Sumpfproduktes beträgt 0,01 Gew.-%.

Der Sumpfaustrag wird einer Propan/Propen-Trennkolonne mit 100 theoretischen Trennstufen zugeführt, die bei 25 bar mit einem Rücklaufverhältnis von 30 betrieben wird. Die Sumpftemperatur beträgt 60 °C, die Kopftemperatur 68 °C. Am Kopf wird Propen mit einem Reinheitsgrad von 99,5 Gew.-% erhalten.

### Vergleichsbeispiel 1

An Stelle der autothermen Propan-Dehydrierung wird eine isotherme Propan-Dehydrierung durchgeführt.

Hierzu wird der Propanstrom auf 450°C vorgeheizt, tritt in die Dehydrierzone ein und wird einer isothermen Dehydrierung unterworfen. Der Wassergehalt des Eintrittsgases beträgt 50 Gew.-%. Der Umsatz der Dehydrierung beträgt, bezogen auf Propan, 50%, die Selektivität der Propen-Bildung beträgt 90%. Daneben werden zu 8% Crack-Produkte und zu 2% CO₂ gebildet. Die Temperatur des Austrittsgases beträgt 600°C.

Die Tabellen 1 (Beispiel) und 2 (Vergleichsbeispiel) geben die Mengen (in kg/h) und Zusammensetzung (in Massenanteilen, Σ = 1,0) der Gasströme gemäß der Figur wieder. Wie diesen entnommen werden kann, ist die Menge an C₃-Kohlenwasserstoffen (Propan und Propen), die mit dem Wasserstoffstrom 9a ausgetragen wird, für das Vergleichsbeispiel sehr viel höher als für das Beispiel.

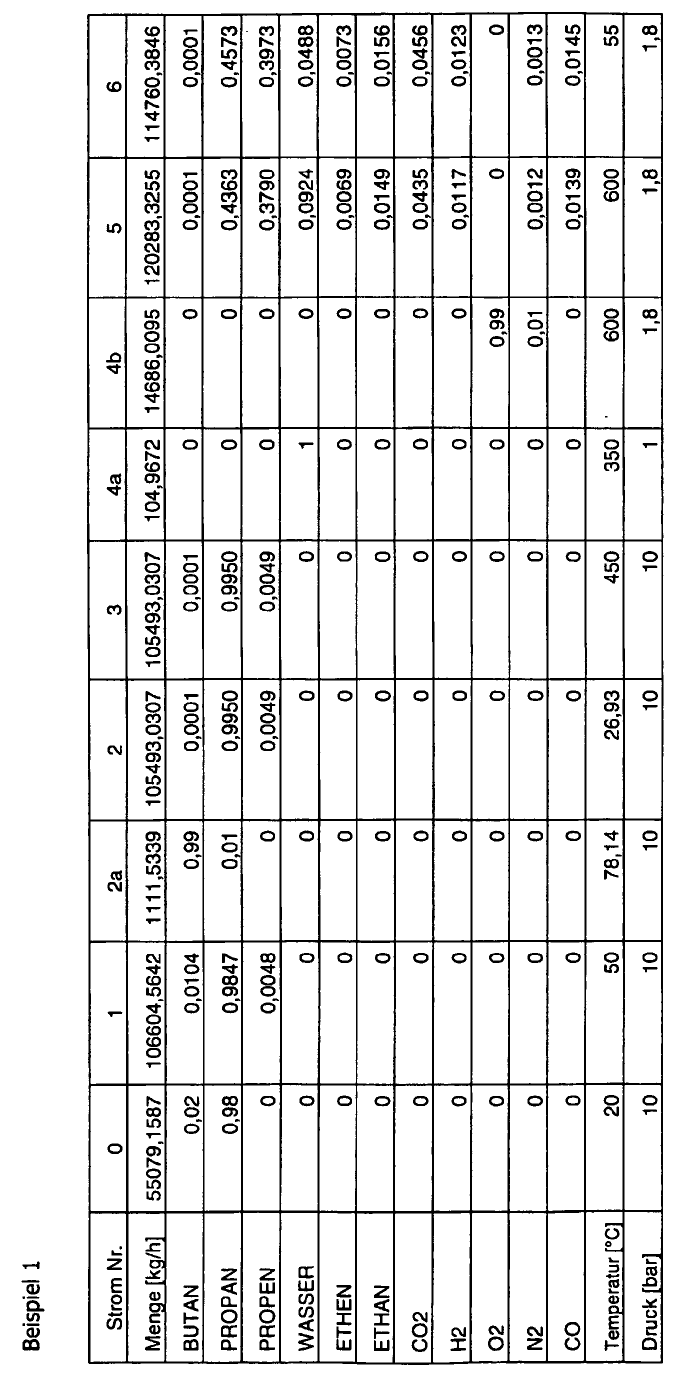

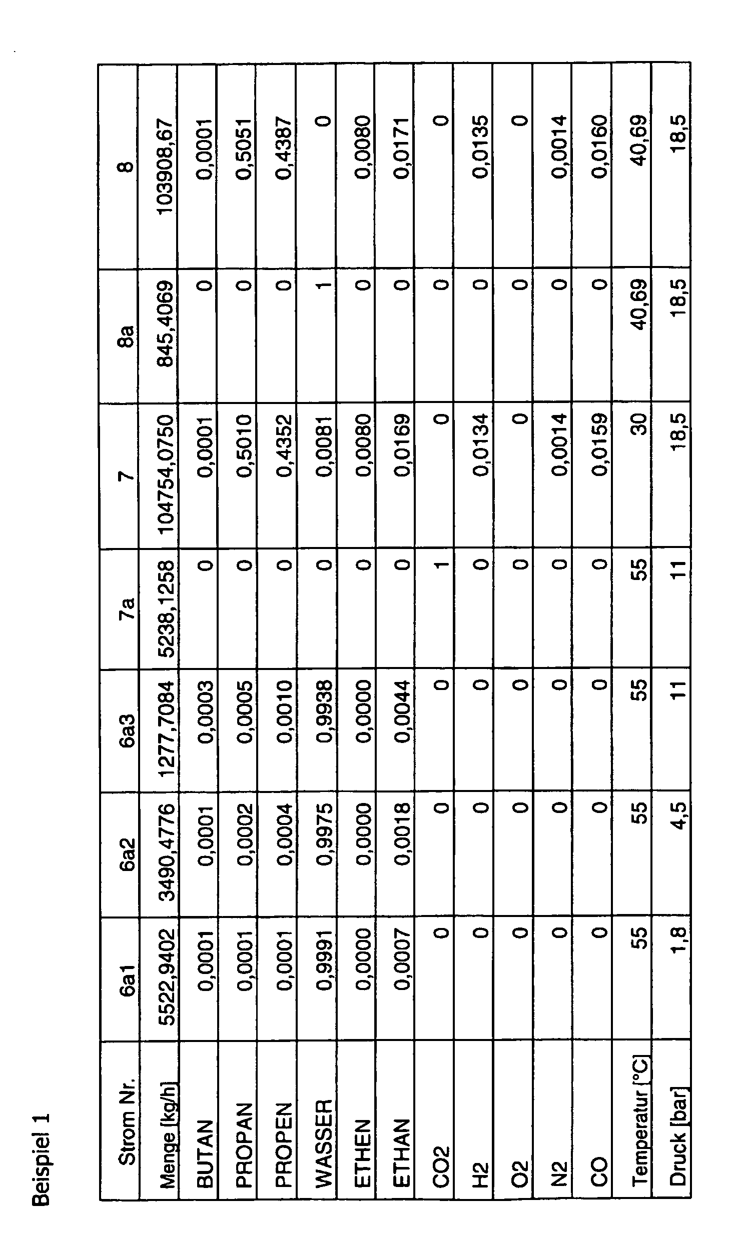

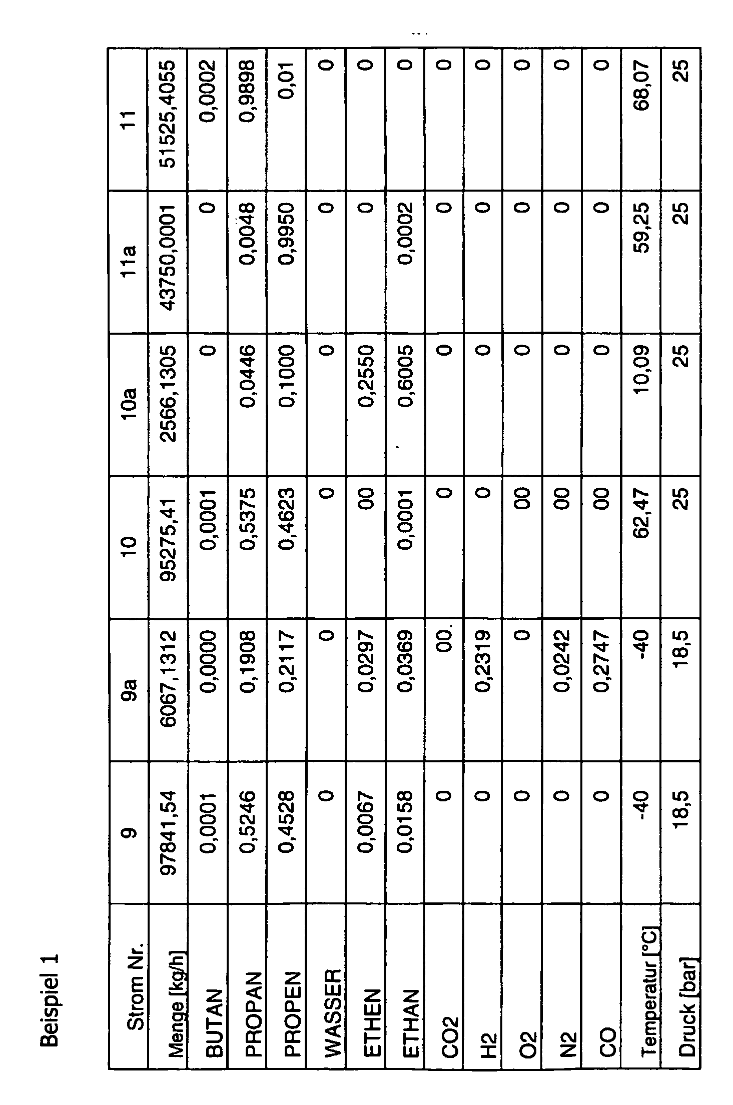

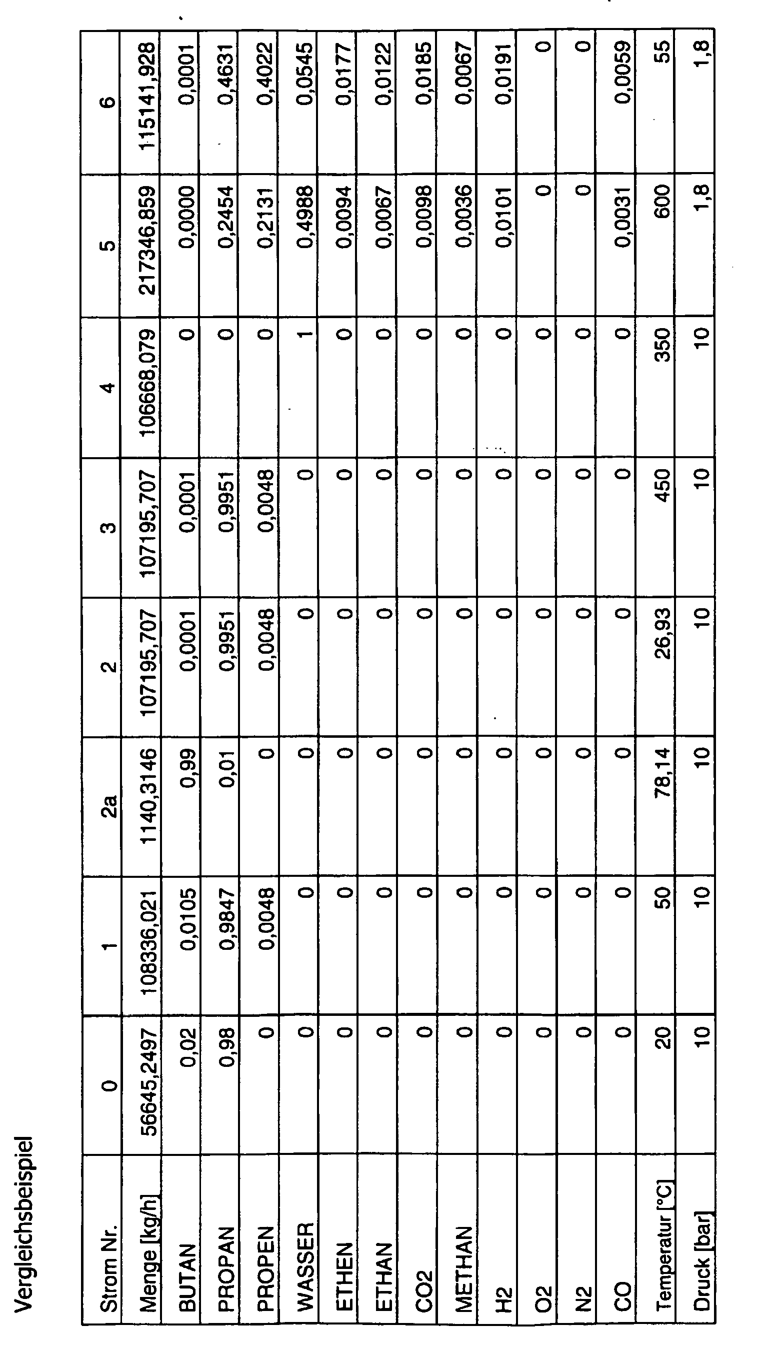

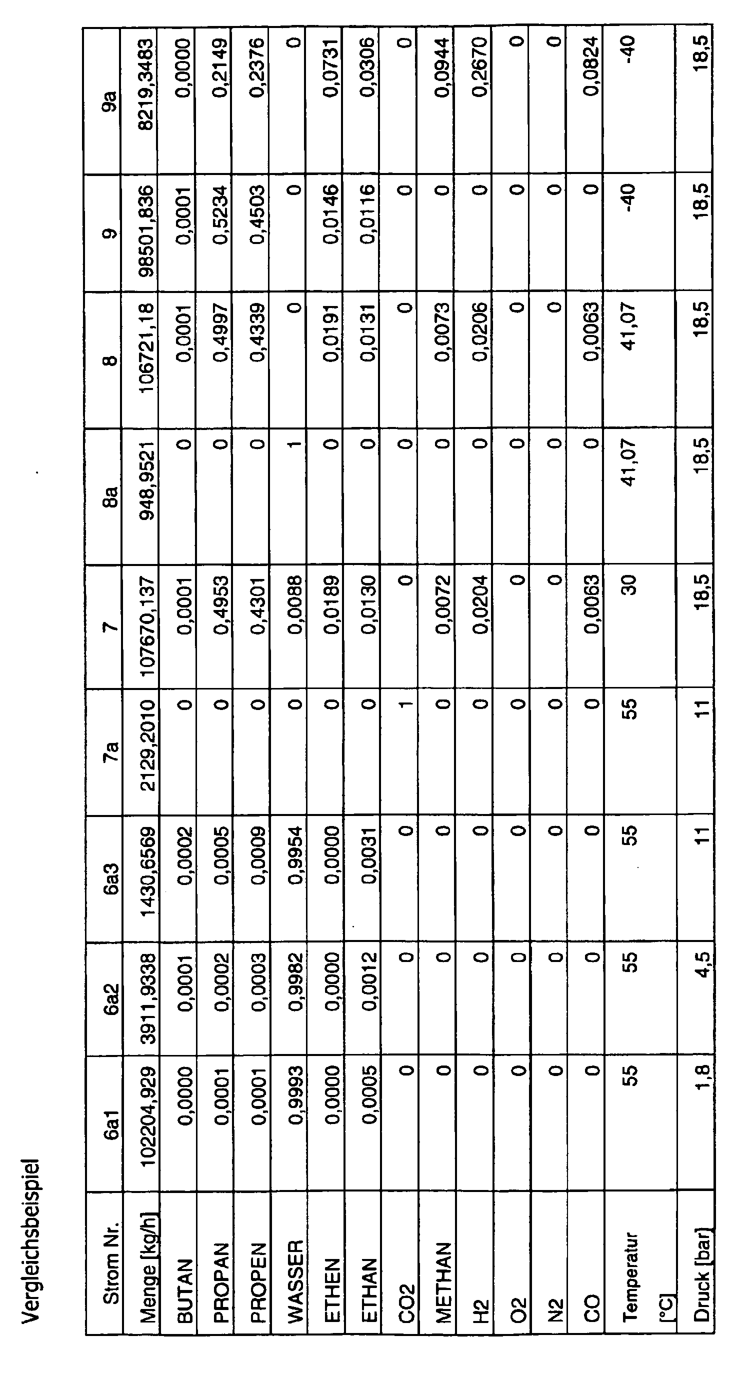

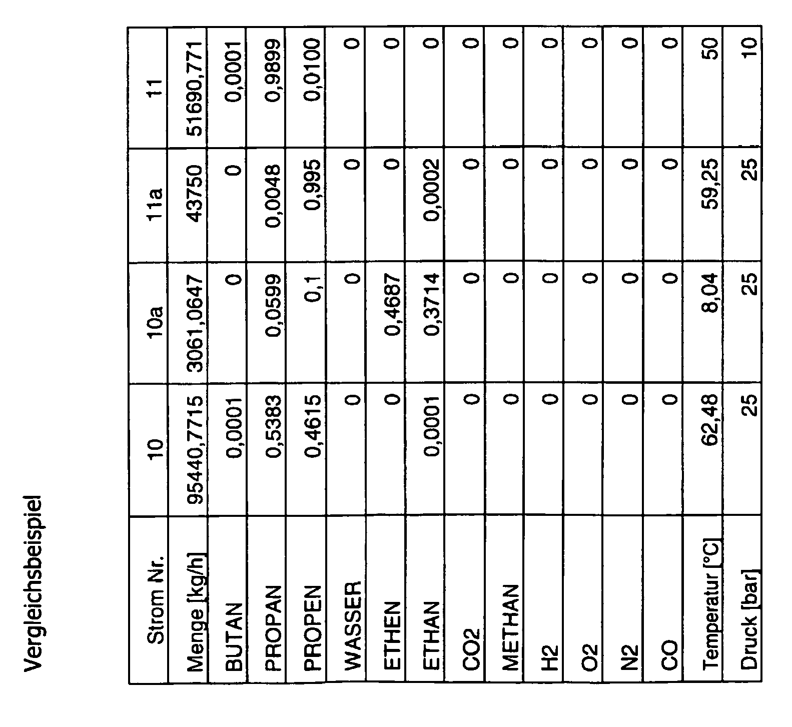

## Patentansprüche

1. Verfahren zur Herstellung von Propen aus Propan mit den Schritten
A) ein Propan enthaltenden Einsatzgasstromes a wird bereitgestellt;
B) der Propan enthaltenden Einsatzgasstromes a und ein sauerstoffhaltiger Gasstrom mit einem Sauerstoff-Gehalt von mindestens 50 Vol.-% werden in eine Dehydrierzone eingespeist und Propan wird einer nicht-oxidativen katalytischen, autothermen Dehydrierung zu Propen unterworfen, wobei ein Produktgasstrom b enthaltend Propan, Propen, Methan, Ethan, Ethen, C₄⁺-Kohlenwasserstoffe, Kohlenmonoxid, Kohlendioxid, Wasserdampf und Wasserstoff erhalten wird,
C) der Produktgasstrom b wird abgekühlt und Wasserdampf durch Kondensierenlassen abgetrennt, wobei ein an Wasserdampf abgereicherter Produktgasstrom c erhalten wird,
D) Kohlendioxid wird durch Gaswäsche abgetrennt, wobei ein an Kohlendioxid abgereicherter Produktgasstrom d erhalten wird,
E) der Produktgasstrom d wird abgekühlt und ein flüssiger Kohlenwasserstoffstroms e1 enthaltend Propan, Propen, Methan, Ethan, Ethen und C₄⁺-Kohlenwasserstoffen durch Kondensierenlassen abgetrennt, wobei ein Restgasstrom e2 enthaltend Methan, Wasserstoff und Kohlenmonoxid verbleibt,
F) der flüssige Kohlenwasserstoffstrom e1 wird in eine erste Destillationszone eingespeist und destillativ in einen Strom f1 enthaltend Propan, Propen und die C₄⁺-Kohlenwasserstoffe und einen Strom f2 enthaltend Ethan und Ethen aufgetrennt,
G) der Strom f1 wird in eine zweite Destillationszone eingespeist und destillativ in einen Produktstrom g1 enthaltend Propen und einen Strom g2 enthaltend Propan und die C₄⁺-Kohlenwasserstoffe aufgetrennt.

2. Verfahren nach Anspruch 1, **dadurch gekennzeichnet, dass** in einem Schritt H) der Strom g2 und frisches Propan in eine dritte Destillationszone eingespeist werden und in der dritten Destillationszone in den Einsatzgasstrom a und einen C₄⁺-Kohlenwasserstoffe enthaltenden Strom destillativ aufgetrennt werden.

3. Verfahren nach Anspruch 1 oder 2, **dadurch gekennzeichnet, dass** in Schritt C) der Produktgasstrom b auf eine Temperatur im Bereich von 30 bis 80 °C abgekühlt wird.

4. Verfahren nach einem der Ansprüche 1 bis 3, **dadurch gekennzeichnet, dass** vor Durchführung von Schritt D) der Produktgasstrom c auf einen Druck von 5 bis 25 bar komprimiert wird.

5. Verfahren nach einem der Ansprüche 1 bis 4, **dadurch gekennzeichnet, dass** vor Durchführung von Schritt E) der Produktgasstrom d auf einen Druck von 5 bis 25 bar komprimiert wird.

6. Verfahren nach einem der Ansprüche 1 bis 5, **dadurch gekennzeichnet, dass** bei Durchführung von Schritt E) der Produktgasstrom d auf eine Temperatur im Bereich von -10 bis -120 °C abgekühlt wird.

7. Verfahren nach einem der Ansprüche 1 bis 6, **dadurch gekennzeichnet, dass** vor Durchführung von Schritt E) der Produktgasstrom d durch Überleiten über ein Molekularsieb getrocknet wird.

8. Verfahren nach einem der Ansprüche 1 bis 7, **dadurch gekennzeichnet, dass** der in Schritt B) eingesetzte sauerstoffhaltige Gasstrom einen Sauerstoffgehalt von mindestens 90 Vol.-% aufweist.

## Claims

1. A process for preparing propene from propane, comprising the steps:
A) a feed gas stream a comprising propane is provided;
B) the feed gas stream a comprising propane and an oxygenous gas stream having an oxygen content of at least 50% by volume are fed into a dehydrogenation zone and propane is subjected to a nonoxidative catalytic, autothermal dehydrogenation to propene to obtain a product gas stream b comprising propane, propene, methane, ethane, ethene, C₄⁺ hydrocarbons, carbon monoxide, carbon dioxide, steam and hydrogen,
C) the product gas stream b is cooled and steam is removed by condensation to obtain a steam-depleted product gas stream c,
D) carbon dioxide is removed by gas scrubbing to obtain a carbon dioxide-depleted product gas stream d,
E) the product gas stream d is cooled and a liquid hydrocarbon stream e1 comprising propane, propene, methane, ethane, ethene and C₄⁺ hydrocarbons is removed by condensation to leave a residual gas stream e2 comprising methane, hydrogen and carbon monoxide,
F) the liquid hydrocarbon stream e1 is fed into a first distillation zone and separated distillatively into a stream f1 comprising propane, propene and the C₄⁺ hydrocarbons and a stream f2 comprising ethane and ethene,
G) the stream f1 is fed into a second distillation zone and separated distillatively into a product stream g1 comprising propene and a stream g2 comprising propane and the C₄⁺ hydrocarbons.

2. The process according to claim 1, wherein, in a step H), the stream g2 and fresh propane are fed into a third distillation zone and separated distillatively in the third distillation zone into the feed gas stream a and a stream comprising C₄⁺ hydrocarbons.

3. The process according to claim 1 or 2, wherein the product gas stream b is cooled in step C) to a temperature in the range from 30 to 80°C.

4. The process according to any of claims 1 to 3, wherein the product gas stream c is compressed to a pressure of from 5 to 25 bar before step D) is carried out.

5. The process according to any of claims 1 to 4, wherein the product gas stream d is compressed to a pressure of from 5 to 25 bar before step E) is carried out.

6. The process according to any of claims 1 to 5, wherein the product gas stream d is cooled to a temperature in the range from -1C to -120°C when step E) is carried out.

7. The process according to any of claims 1 to 6, wherein the product gas stream d is dried by passing over a molecular sieve before step E) is carried out.

8. The process according to any of claims 1 to 7, wherein the oxygenous gas stream used in step B) has an oxygen content of at least 90% by volume.

## Revendications

1. Procédé de fabrication de propène à partir de propane par les étapes suivantes :
A) un courant gazeux d'entrée a contenant du propane est préparé ;
B) le courant gazeux d'entrée a contenant du propane et un courant gazeux contenant de l'oxygène ayant une teneur en oxygène d'au moins 50 % en volume sont introduits dans une zone de déshydrogénation et le propane est soumis à une déshydrogénation autotherme, catalytique et non oxydative en propène, un courant gazeux produit b qui contient du propane, du propène, du méthane, de l'éthane, de l'éthène, des hydrocarbures en C₄⁺, du monoxyde de carbone, du dioxyde de carbone, de la vapeur d'eau et de l'hydrogène étant obtenu,
C) le courant gazeux produit b est refroidi et la vapeur d'eau est séparée par condensation, un courant gazeux produit c appauvri en vapeur d'eau étant obtenu, D) le dioxyde de carbone est séparé par épuration gazeuse, un courant gazeux produit d appauvri en dioxyde de carbone étant obtenu,
E) le courant gazeux produit d est refroidi et un courant liquide d'hydrocarbures e1 qui contient du propane, du propène, du méthane, de l'éthane, de l'éthène et des hydrocarbures en C₄⁺ est séparé par condensation, un courant gazeux résiduel e2 qui contient du méthane, de l'hydrogène et du monoxyde de carbone demeurant,
F) le courant liquide d'hydrocarbures e1 est introduit dans une première zone de distillation et séparé par distillation en un courant f1 qui contient du propane, du propène et les hydrocarbures en C₄⁺ et un courant f2 qui contient de l'éthane et de l'éthène,
G) le courant f1 est introduit dans une deuxième zone de distillation et séparé par distillation en un courant produit g1 qui contient du propène et un courant g2 qui contient du propane et les hydrocarbures en C₄⁺ .

2. Procédé selon la revendication 1, **caractérisé en ce qu'**à l'étape H), le courant g2 et du propane frais sont introduits dans une troisième zone de distillation et séparés par distillation dans la troisième zone de distillation pour former le courant gazeux d'entrée a et un courant contenant des hydrocarbures en C₄⁺.

3. Procédé selon la revendication 1 ou 2, **caractérisé en ce qu'**à l'étape C), le courant gazeux produit b est refroidi à une température dans la plage allant de 30 à 80 °C.

4. Procédé selon l'une quelconque des revendications 1 à 3, **caractérisé en ce qu'**avant la réalisation de l'étape D), le courant gazeux produit c est comprimé à une pression de 5 à 25 bars.

5. Procédé selon l'une quelconque des revendications 1 à 4, **caractérisé en ce qu'**avant la réalisation de l'étape E), le courant gazeux produit d est comprimé à une pression de 5 à 25 bars.

6. Procédé selon l'une quelconque des revendications 1 à 5, **caractérisé en ce que** lors de la réalisation de l'étape E), le courant gazeux produit d est refroidi à une température dans la plage allant de -10 à -120 °C.

7. Procédé selon l'une quelconque des revendications 1 à 6, **caractérisé en ce qu'**avant la réalisation de l'étape E), le courant gazeux produit d est séché par passage sur un tamis moléculaire.

8. Procédé selon l'une quelconque des revendications 1 à 7, **caractérisé en ce que** le courant gazeux contenant de l'oxygène utilisé à l'étape B) présente une teneur en oxygène d'au moins 90 % en volume.
